Europäisches Patentamt

European Patent Office

Office européen des brevets

(19)

(11) **EP 1 262 551 A2**

(12) **EUROPEAN PATENT APPLICATION**

(43) Date of publication:
 **04.12.2002 Bulletin 2002/49**

(51) Int Cl.⁷: **C12N 15/53**, C12N 9/04,
 C12N 15/63, C12N 1/21

(21) Application number: **02253771.6**

(22) Date of filing: **29.05.2002**

| | |
|---|---|
| (84) Designated Contracting States:<br>**AT BE CH CY DE DK ES FI FR GB GR IE IT LI LU<br>MC NL PT SE TR**<br>Designated Extension States:<br>**AL LT LV MK RO SI** | (72) Inventors:<br>• **Masuda, Ikuko**<br>  **Noda-shi, Chiba 278-8601 (JP)**<br>• **Ichikawa, Toshio**<br>  **Noda-shi, Chiba 278-8601 (JP)** |
| (30) Priority: **29.05.2001 JP 2001159870** | (74) Representative:<br>**Denison, Christopher Marcus et al** |
| (71) Applicant: **KIKKOMAN CORPORATION**<br>**Noda-shi, Chiba 278-8601 (JP)** | **Mewburn Ellis**<br>**York House**<br>**23 Kingsway**<br>**London WC2B 6HP (GB)** |

(54) **A sorbitol dehydrogenase gene, a recombinant DNA, and a process for producing sorbitol dehydrogenase**

(57)    A sorbitol dehydrogenase gene encoding (a) a protein having an amino acid sequence shown in SEQ ID NO:1; or (b) a protein with sorbitol dehydrogenase activity consisting of an amino acid sequence comprising one or more deletions, substitutions or additions in the amino acid sequence of (a). Further, the invention provides a novel recombinant DNA having the sorbitol dehydrogenase gene inserted into a vector DNA, and a transformant or a transductant containing the recombinant DNA. Also, the invention provides a sorbitol dehydrogenase production method comprising the steps of: culturing the transformant or the transductant in a medium; and collecting sorbitol dehydrogenase from the culture. According to the present invention, sorbitol dehydrogenase can be produced efficiently.

EP 1 262 551 A2

**Description**

This invention relates to a gene of sorbitol dehydrogenase which produces D-fructose by oxidizing D-sorbitol in the presence of NAD+ and which produces, through a reverse reaction of the above reaction, D-sorbitol by reducing D-fructose in the presence of NADH, a novel recombinant DNA, and a method for producing sorbitol dehydrogenase.

D-sorbitol exists in trace amounts in blood serum, urine or the like of humans, and it is known that its content is an important indicator in the diagnosis of diseases, for example, diabetes. Now at research institutes and so on, an enzyme method is used for the determination of D-sorbitol in blood serum.

Further, by hydrogenating glucose under high temperature and pressure, D-sorbitol solution is obtained, and then D-sorbitol powder or granule is obtained by refining the obtained D-sorbitol solution by use of ion-exchange resin method or the like. D-sorbitol is odorless but it has a brisk sweetness, and thereby brings coolness to the tongue or it has an excellent hygroscopicity. Due to these properties, it is widely used as a sweetener for chewing gum, candy, and other confectionary, as a robustness-imparting material for synthetic alcohol, or as a sweetener and humectant for tobacco. In addition, D-fructose can be obtained by methods such as a method of hydrolyzing a plant like *Heliantus tuberosus*, which contains inulin in abundance, and a method of converting sucrose by the action of invertase. D-fructose has a delicate but strong sweetness, and imparts wettability. Thus, it is widely used as a sweetener or the like in the food industry.

In view of the foregoing, it is of industrially important significance to obtain an enzyme usable for determining D-sorbitol existing in trace amounts in blood serum, urine or the like, and D-sorbitol or D-fructose contained in foods.

Sorbitol dehydrogenase has conventionally been produced, for instance by inoculating *Pseudomonas sp.* in a medium, culturing it, and collecting the culture product. (refer to Japanese Patent No. 3152855)

However, the above production method of sorbitol dehydrogenase has some drawbacks such as insufficient yield.

In order to address these problems of the prior art, it is an object of the present invention to provide a sorbitol dehydrogenase gene, a novel recombinant DNA thereof, and a method for producing sorbitol dehydrogenase.

As a result of various reviews on the above problems, the inventors of the present invention have successfully accomplished isolation of a sorbitol dehydrogenase gene derived from *Pseudomonas sp.* KS-E1806, and determination of the structure thereof. Further, the inventors have obtained a recombinant DNA having a gene encoding sorbitol dehydrogenase inserted into a vector DNA, and it has been found that after this recombinant DNA is incorporated into a strain belonging to the genus *Escherichia,* and the recombinant-DNA-containing strain with sorbitol dehydrogenase production ability is cultured, sorbitol dehydrogenase can effectively be produced. Based on these findings, the inventors accomplished the present invention.

In accordance with a first aspect of the present invention, there is provided a sorbitol dehydrogenase gene encoding the following protein (a) or (b):

(a) a protein having an amino acid sequence shown in SEQ ID NO:1;or
(b) a protein which consists of an amino acid sequence having one or more amino acid deletions, substitutions or additions relative to the amino acid sequence of (a), and has sorbitol dehydrogenase activity.

In accordance with a second aspect of the present invention, there is provided a sorbitol dehydrogenase gene comprising the following DNA (a) or (b):

(a) a DNA having a nucleotide sequence shown in SEQ ID NO:2; and
(b) a DNA encoding a protein which hybridizes with the DNA having a nucleotide sequence complementary to the DNA having a nucleotide sequence of (a) under stringent conditions, and has sorbitol dehydrogenase activity.

In accordance with a third aspect of the present invention, there is provided a novel recombinant DNA wherein the above sorbitol dehydrogenase gene has been inserted into a vector DNA.

In accordance with a fourth aspect of the present invention, there is provided a transformant or transductant including the above novel recombinant DNA.

In accordance with a fifth aspect of the present invention, there is provided a method for producing sorbitol dehydrogenase, comprising the steps of:

culturing the transformant or the transductant in a medium; and
collecting sorbitol dehydrogenase from the medium.

The invention, and preferred embodiments, will now be described in detail.

For example, a sorbitol dehydrogenase gene of the present invention can be isolated as follows.

First, *Pseudomonas sp.* KS-E1806 is cultivated, e.g. by a method described in Japanese Patent No. 3152855, and then chromosomal DNA is extracted from the obtained microorganism strain. The chromosomal DNA can be prepared from cell bodies of the strain, e.g. by a method described in Current Protocols in Molecular Biology (WILEY Interscience, 1989).

Next, the above microorganism is cultivated e.g. by a method described in Japanese Patent No. 3152855, and sorbitol dehydrogenase is purified, thereby obtaining sorbitol dehydrogenase.

The obtained sorbitol dehydrogenase is subjected to lysylendopeptidase (available from Wako Pure chemical Industries, Ltd.) treatment under a degeneration condition for fragmentation. The above fragment is fractionated, for example, by reverse phase high performance liquid chromatography using Capsulepack C18UG300 column (Shiseido Co., Ltd.), and amino acid sequences with regard to several kinds of fragment peptides are determined, e.g. by Procise 492 protein sequencer (manufactured by Applied Biosystems Japan Ltd.).

Based on the above amino acid sequences, a primer for polymerase chain reaction (hereinafter abbreviated as PCR) is prepared. At this time, considering codon degeneracy, while a nucleotide mixture is used at a position where there is a codon degeneracy of not greater than two kinds of nucleotides, inosine is used at a position where there is a codon degeneracy of 3 to 4 kinds of nucleotides. Then, a long primer with a length of not less than 40 mer, preferably 42 to 44 mer, is prepared. PCR is conducted with the prepared primer using chromosomal DNA of the previously obtained *Pseudomonas sp.* KS-E1806 as a template. The amplified DNA fragment is incorporated into a vector DNA, such as pCR2.1 vector (available from Invitorogen Japan K.K.), affording a recombinant plasmid. A nucleotide sequence of the inserted DNA in the plasmid is determined, e.g. by a Multi Capirally DNA ayalysis system CEQ2000 (manufactured by Beckman Coulter, Inc.), then a DNA having at both ends a nucleotide sequence correctly encoding the amino acid sequence of the peptide used for designing PCR primer is selected. The thus obtained amplified DNA fragment is a part of the sorbitol dehydrogenase gene of the present invention (partial gene).

The above partial gene is labeled, and a clone containing a target gene is isolated by hybridization from a library containing the chromosomal fragment of *Pseudomonas sp.* KS-E1806. DNA fragment labeling and hybridization detection can be carried out, e.g. by DIG system (manufactured by Roche Diagnostics K. K.).

The library can be constructed, for example, as follows. First, the chromosomal DNA is completely digested by a restriction enzyme such as EcoRI, and then subjected to conventional agarose gel electrophoresis and Southern blot analysis using the above PCR product as a probe. As a result, the chain length of the DNA fragment containing sorbitol dehydrogenase gene is determined.

Next, chromosomal DNA is completely digested by a restriction enzyme above such as EcoRI, and subjected to agarose gel electrophoresis, thereafter a gel containing the determined chain length of DNA fragment is excised. Then, DNA in the excised gel is collected by GENE CLEAN II (manufactured by Funakoshi Co., Ltd.), and incorporated into a vector DNA in accordance with a conventional method. Examples of the vector DNA to be used include a plasmid DNA such as pUC119 (available from Takara Shuzo Co., Ltd.) and pBR322 (available from Takara Shuzo Co., Ltd.), and a bacteriophage DNA such as λENBL3 (available from Stratagene) and λDASH II (available from Funakoshi Co., Ltd.).

By use of the recombinant DNA thus obtained, for example, *Escherichia coli* K-12, preferably *Escherichia coli* JM109 (available from Takara Shuzo Co., Ltd.), DH5α (available from Takara Shuzo Co., Ltd.) is transformed, or the recombinant DNA is transduced thereinto, so as to obtain a transformant or a transductant. Examples of a host cell to be used, in addition to a cell of *Escherichia coli*, include yeast, a fungus, actinomyces, and an animal cell.

This transformation can be carried out, e.g. by a method of D. M. Morrison (Method in Enzymology, 68, 326-331, 1979). Further, the transduction can be carried out, e.g. by a method of B. Hohn (Method in Enzymology, 68, 299-309, 1979).

In order to obtain a purified recombinant plasmid DNA from the isolated transformant (containing sorbitol dehydrogenase gene therein), QIAGEN Plasmid Midi Kit (available from Qiagen K.K.), for example, can be used.

By using the purified recombinant plasmid DNA, the whole nucleotide sequence of sorbitol dehydrogenase gene is analyzed by means of a method explained in process (5) of the Examples described hereinafter, then determining amino acid sequence of the polypeptide translated from the gene having the above nucleotide sequence therein.

This amino acid sequence is as shown in SEQ ID NO:1. The sorbitol dehydrogenase gene of the present invention is a gene encoding the amino acid sequence thus determined.

Incidentally, as far as a sorbitol dehydrogenase gene encodes an amino acid sequence comprising one or more, preferably several amino acid deletions, substitutions, or additions relative to the amino acid sequence shown in SEQ ID NO:1, and having sorbitol dehydrogenase activity, such a sorbitol dehydrogenase gene is included in the present invention.

Moreover, as far as there is obtained a sorbitol dehydrogenase gene encoding an amino acid sequence comprising one or more amino acid deletions, substitutions, or additions relative to the amino acid sequence shown in SEQ ID NO:1, and having a sorbitol dehydrogenase activity, any method may be applied therefor. Examples thereof include site-specific mutation induction which is a well-known method to cause a point mutation or a deletion mutation on a gene; a method comprising of selectively cleaving a gene, deleting or adding a selected nucleotide, and ligating the gene; and an oligonucleotide mutation induction method.

These DNAs are highly likely to encode a polypeptide having sorbitol dehydrogenase activity, and it is possible to produce transformants and select transformants having activity.

In order to obtain a gene substantially identical to the sorbitol dehydrogenase gene of the present invention, DNA encoding a polypeptide having sorbitol dehydrogenase activity can be selected by performing hybridization under stringent conditions with a DNA having the nucleotide sequence of SEQ ID NO:2, a chain complementary thereto, or a probe containing a fragment thereof. Herein, stringent conditions mean conditions wherein only a specific hybrid is selectively formed, enabling signal detection, yet a non-specific hybrid is not produced. Although such conditions are slightly different depending on individual biological species, conditions can easily be determined by examining several salt concentrations or temperatures for hybridization, and washing by means of a conventional method. As these conditions, for example, since a specific signal is observed in items (3) and (4) of the Examples described hereinafter, the hybridization is conducted at a temperature of 37 to 42 °C overnight by use of DIG Easy Hyb reagent (available from Roche Diagnostics K. K.). The washing is carried out twice with $0.5 \times$ SSC, 0.1 % SDS for 15 minutes. The washing is carried out at a temperature of not less than 45 °C, preferably not less than 52 °C, more preferably not less than 57 °C. Although a DNA which can be hybridized under such conditions is highly likely to encode a peptide having sorbitol dehydrogenase activity, DNA containing a variation causing a loss of sorbitol dehydrogenase activity may be included. However, such variation-containing DNA can be removed, after transformation, by determining the sorbitol dehydrogenase production ability of the transformant.

The recombinant DNA thus obtained containing a sorbitol dehydrogenase gene does not have a promoter for attaining a strong expression in *E. coli*, and thus the transformant strain having the recombinant DNA has a low productivity of sorbitol dehydrogenase. Then, a strain with a high productivity of sorbitol dehydrogenase is obtained by the following operation.

First, based on the nucleotide sequence obtained in the above operation, oligonucleotides comprising N-terminus and the C-terminus of sorbitol dehydrogenase gene, respectively, and about 12 nucleotides before and after N- or C- terminus (the oligonucletide comprising in total 30 nucleotides; the oligonucleotide at C-terminus is a complementary strand) are synthesized, and the synthesized oligonucleotides are used as primers. NdeI sites have been incorporated into the synthesized primers such that a coding region is obtained after amplified product by PCR is digested by application of NdeI (available from Takara Shuzo Co., Ltd.). In other words, coding region of sorbitol dehydrogenase can be obtained by carrying out PCR using the purified recombinant plasmid DNA obtained above as a template followed by digesting the resulting product by NdeI.

The obtained DNA is inserted into a vector DNA having a DNA sequence containing an expression region such as a promoter, an operator, and a ribosome binding site derived from *E. coli* lactose operon (See The Operon, p. 227, Cold Spring Harbor Laboratory, 1980). The vector DNA to be used may be a plasmid DNA or a bacteriophage DNA. For example, a vector pUTE500K' (disclosed in Japanese Patent Application Laying-Open (kokai) No. 08-205861) described in process (6) of Examples can be used. Using the obtained recombinant DNA, for example, *E. coli* K-12, preferably *E. coli* JM109 (available from Takara Shuzo Co., Ltd.), or DH5$\alpha$ (available from Takara Shuzo Co., Ltd.), is transformed, or the recombinant DNA is transduced thereinto, thereby each affording a strain.

The production of sorbitol dehydrogenase by use of the above obtained transformant or transductant, for example, a strain belonging to the genus *Escherichia*, each having the ability for producing a sorbitol dehydrogenase, can be carried out as follows. For culturing the above microorganism, a conventional solid culture method may be used. However, it is preferable to use a liquid culture method for culturing as much as possible.

Additionally, as a medium for culturing the above microorganism, there may be used a medium prepared by adding at least one mineral salt, such as potassium dihydrogenphosphate, dipotassium hydrogenphosphate, magnesium sulfate, ferric chloride, ferric sulfate, and manganese sulfate; further if necessary, carbohydrate, vitamin, or the like; to at least one nitrogen source, such as a yeast extract, peptone, meat extract, corn steep liquor, or an exudation of soybean or wheat malt.

Further, it is appropriate to adjust initial pH of the medium to 7 to 9. Further, the culture is conducted for 6 to 24 hours at a temperature of from 30 to 42 °C, preferably about 37 °C by submerged culture with aeration-agitation, shake-culture, stationary culture, or the like. After the culture is finished, a conventional enzyme collection means may be used for collecting sorbitol dehydrogenase from the culture product.

Bacterial bodies are separated from the culture product, for example, by filtration, centrifugation or the like, and washed. It is preferred to collect sorbitol dehydrogenase from the bacterial bodies. In this case, the bacterial bodies per se may be used. However, it is preferable to collect sorbitol dehydrogenase from the bacterial bodies by a method of disrupting the bacterial bodies by using a disrupting means such as a ultrasonic disruptor, French press, and a dynamill, a method of digesting cell wall of the bacterial bodies by using cell wall lysing enzyme such as lysozyme, and a method of extracting an enzyme from the bacterial bodies by using a surfactant such as Triton X-100.

For isolating sorbitol dehydrogenase from the crude enzyme solution thus obtained, a conventional method used for enzyme purification may be used. Examples of conventional methods include ammonium sulfate salting-out, organic solvent precipitation, ion-exchange chromatography, gel filtration chromatography, adsorption chromatography, electrophoresis, and these may preferably be used in combination as required.

The obtained sorbitol dehydrogenase has physico-chemical properties as shown below.

(1) Function: to oxidize, in the presence of NAD+, D-sorbitol to produce D-fructose and NADH, and by the reverse reaction thereof, to reduce, in the presence of NADH, D-fructose to produce D-sorbitol and NAD+.

(2) Substrate specificity: to react specifically to D-sorbitol and galactitol.

(3) Optimum pH and stable range of pH: an optimum pH is about 10, and a stable pH range is from 5.5 to 10.5 in the case of 4-hour treatment at 30 °C.

(4) Optimum reaction temperature range: in the case of 100 mM Tris-HCl buffer solution (pH 9.0), the optimum reaction temperature range is about 50 °C.

(5) Deactivation condition by pH, temperature, etc.: in the case of 4-hour treatment at 30 °C, sorbitol dehydrogenase is kept stable at pH of 5.5 to 10.5, and at a pH of not greater than 5.0 and not less than 11.0 the sorbitol dehydrogenase is completely deactivated. In the case of 30-minute treatment at pH of 7.0, sorbitol dehydrogenase is kept stable at a temperature of up to around 40 °C. In case of 40 °C treatment at pH of 7.0, 24 hours are enough for complete deactivation.

(6) Inhibition: $HgCl_2$ has a strong inhibitory action.

(7) Molecular weight: about 64,500±5,000 (gel filtration)

(8) Assay of titer: the titer of the enzyme is assayed by the following method, and an amount of the enzyme for producing 1 $\mu$mol of NADH for one minute is taken as 1U.

(Preparation of reagents)

First solution; substrate solution, 20 g of D-sorbitol is dissolved in distilled water, and filled to 100 ml.

Second solution; substrate solution, 86 mg of NAD+ is dissolved in distilled water, and filled to 4 ml.

Third solution; buffer solution, 6.05 g of tris(hydroxymethyl)aminomethane is dissolved in distilled water, and pH is adjusted to 9.0 by 4N HCl, then filled to 500 ml.

(Determination procedure)

1) 0.5 ml of the first solution, 0.05 ml of the second solution, and 2.4 ml of the third solution are mixed with each other and pre-incubated for 5 minutes at 37 °C.

2) The 5-minute pre-incubated solution and 0.05 ml of an enzyme solution adjusted to 0.1 to 0.8 U/ml are mixed with each other, and the increased value of absorbance of a sample per 1 minute is measured at 37 °C at a wavelength of 340 nm.

3) For determination of a blank value, 0.5 ml of distilled water is added instead of the first solution, and 0.05 ml of the second solution and 2.4 ml of the third solution are mixed therewith, and pre-incubated for 5 minutes at 37 °C. Then, 0.05 ml of the enzyme solution adjusted to 0.1 to 0.8 U/ml are mixed with the pre-incubated solution, and the increased value of absorbance of the blank per 1 minute is measured at 37 °C at a wavelength of 340 nm.

(Calculation of titer value)

$$U/ml= [\{(\text{change of absorbance value of sample - increase of absorbance value of blank}) \times 3\} / (6.22 \times 0.05) ] \times \text{dilution factor}$$

The enzyme acts on D-sorbitol, and has stable enzyme activity even under conditions, which are often applied for measurement according to a conventional enzyme method (reaction at pH 9.0, 37 °C, for 30 minutes). By use of the enzyme, for example, the amount of D-sorbitol present in human blood serum or urine, or foods can be determined with excellent accuracy. Further, by the reverse reaction of the enzyme, for example, the amount of D-fructose in foods can be determined.

EXAMPLES

Hereinafter, the present invention is described in more detail by reference to the Examples, which, however, are not intended to limit the scope of the present invention.

(1) Preparation of a chromosomal DNA of *Pseudomonas sp.* KS-E1806

*Pseudomonas sp.* KS-E1806 (FERM BP-7616) (converted from FERM P-14299) was inoculated onto 100 ml of a enzyme production medium [0.5 % of D-sorbitol, 2.0 % of polypepton, 0.5 % of yeast extract, 0.01 % of $KH_2PO_4$, 0.01 % of $K_2HPO_4$, 0.01 % of $MgSO_4 \cdot 7H20$, and tap water (at pH of 7.2)], and shake-cultured at 30 °C for about 20 hours, then collecting bacterial bodies by centrifugation. From the bacterial bodies 1.2 mg of chromosomal DNA was obtained by use of G NOME DNA Isolation Kit (available from Funakoshi Co., Ltd.).

(2) Acquisition of partial gene

Next, sorbitol dehydrogenase was purified from the above microorganism by a method described in Japanese Patent No. 3152855, and 50 µg of the obtained sorbitol dehydrogenase was subjected to lysylendopeptidase (Wako Pure Chemical Industries, Ltd.) treatment under denaturing conditions, resulting in fragmentation. The resultant fragments were fractionated and collected by reversed phase HPLC using Waters µBondapack C18 (available from Millipore Corporation), then amino acid sequences of several kinds of fragmental peptides were determined by use of Procise 492 protein sequencer (manufactured by Applied Biosystems Japan Ltd.).

In accordance with the above internal amino acid sequence, a plurality of PCR primers were prepared, and PCR was conducted by use of ExTaq DNA polymerase (available from Takara Shuzo Co., Ltd.) with the chromosomal DNA prepared in process (1) used as a template. First, 15-26 mer primers were plurally prepared by using the nucleotide mixture at a position where there is a codon degeneracy. Although PCR was conducted with various combinations of these primers, annealing temperatures being reviewed, a target partial gene could not be obtained. Therefore, long primers, in which a sense primer is 42mer [an internal amino acid sequence of the obtained protein: a polynucletide corresponding to Val Asn Gly Ile Ala Pro Gly Val Val Asp Thr Pro Met Trp: 5'-GTIAAYGGIATIGCICCIGGIGTIGTITGYACICCIATGTGG-3' (A: adenine, C: cytosine, G: guanine, T: thymine, I: inosine, Y: thymine or cytosine)] and an antisense primer is 44mer mer [an internal amino acid sequence of the obtained protein: a polynucletide corresponding to Asp Ala Asp Tyr Ile Thr Ala Gln Thr Leu Asn Val Asp Gly Gly: 5'-CCICCRTCIACRTTIAIIGTYTGIGCIGTIATRTARTCIGCRTC-3' (A: adenine, C: cytosine, G: guanine, T: thymine, I: inosine, R: adenine or guanine, Y: thymine or cytosine)], were then prepared by using the nucleotide mixture at a position where there is a codon degeneracy of one or two kinds of nucleotides, and inosine at a position where there is a codon degeneracy of three to four kinds of nucleotides. By use of the prepared primers, annealing at 64 °C and 60 cycles of PCR reactions were conducted, and subsequently a plurality of gene fragments were amplified.

Among them, four major DNA fragments were incorporated into pCR2.1 vector (available from Invitrogen Japan K. K.), so that a recombinant plasmid was obtained. A nucleotide sequence of the inserted DNA in the plasmid was determined by a multi capillary DNA analysis system CEQ2000 (manufactured by Beckman Coulter, Inc.). As a result, the longest DNA fragment (227 bp) of the four fragments had at both ends thereof nucleotide sequences correctly encoding amino acid sequences of the peptides used for designing the PCR primers. In addition, in the amino acid sequence deduced based on the determined nucleotide sequence, there existed the internal amino acid sequence determined by the protein sequencer. Accordingly, it was proved the amplified DNA fragment thus obtained was a part of the sorbitol dehydrogenase gene (partial gene) of the present invention.

(3) Construction of chromosomal DNA library

First, 2 µg of the chromosomal DNA were completely digested by EcoRI, and thereafter subjected to a conventional agarose gel electrophoresis, further to Southern blot analysis with the above 227 bp PCR product as a probe. The probe was labeled with digoxigenin using DIG-High Prime (available from Roche Diagnostics K. K.). As a result, it was confirmed that the EcoRI fragment containing a sorbitol dehydrogenase gene had a chain length of about 1.3 kb. Then, 10 µg of the chromosomal DNA was completely digested by a restriction enzyme EcoRI, and subjected to agarose gel electrophoresis.

Next, after excising gel containing the EcoRI fragment with a chain length of 1.3 kb, DNA in the gel was collected by GENE CLEAN II (manufactured by Funakoshi Co., Ltd.). The DNA and EcoRI digest of 100ng of a plasmid vector DNA pUC19 (available from Takara Shuzo Co., Ltd.) were ligated with one unit of T4 DNA Ligase (available from Roche Diagnostics K. K.). The obtained recombinant plasmid DNA was used for transforming *Escherichia coli* DH5α strain (available from Takara Shuzo Co., Ltd.) in accordance with a method of D.M. Morrison (Methods in Enzymology, 68, p. 326-331, 1979). As a consequence, 2000 colonies were obtained, and blotted on a nylon membrane filter Hybond-N+ (available from Amersham Pharmacia Biotech K. K.) according to the protocol of Amersham Pharmacia Biotech K. K.

(4) Isolation of sorbitol dehydrogenase gene by colony hybridization

A sorbitol dehydrogenase gene was obtained by screening the chromosomal DNA library constructed in process (3) with 227 bp PCR product obtained in process (2) as a probe. DIG High Prime (available from Roche Diagnostics K. K.) was used to label the probe with digoxigenin. In the screening, colony hybridization was conducted, in accordance with a method described in Current Protocols in Molecular Biology (WILEY Interscience, 1988), thereby obtaining four positive colonies. From the four positive colonies, plasmid DNA was prepared by QIAGEN Plasmid Midi Kit (available from Qiagen K. K.).

(5) Analysis of sorbitol dehydrogenase gene

With respect to the EcoRI fragment inserted into the plasmid DNA obtained from the four positive colonies, about 200 bp of nucleotide sequences from both ends thereof were analyzed by Multi Capillary DNA Analysis System CEQ2000 (manufactured by Beckman Coulter, Inc.), and thereafter all of them were considered the same clone. Results of determining the entire nucleotide sequence of the inserted fragments showed that the full length of a sorbitol dehydrogenase gene was included. The determined nucleotide sequence of the sorbitol dehydrogenase gene, and the amino acid sequence of the polypeptide translated from the DNA sequence are shown in SEQ ID NO:2 and SEQ ID NO:1, respectively. It was proved that an ORF of the sorbitol dehydrogenase gene comprises 774 bp, 258 amino acids.

(6) Preparation of *Escherichia coli* DH5α (pSDH1)

A DNA consisting of a coding region for soribtol dehydrogenase was prepared by the following method. Prepared as PCR primers were an oligonucleotide comprising the N-terminus of the sorbitol dehydrogenase gene and about 12 nucleotides each before and after the N-terminus (SEQ ID NO:3) and an oligonucleotide comprising the C-terminus thereof and about 12 nucleotides each before and after the C-termius (SEQ ID NO:4). These primers were designed to have an NdeI site incorporated in the sequence thereof such that the coding region is obtained by digesting a PCR amplified product with NdeI.

Using these primers, PCR was conducted with the plasmid DNA obtained from the positive colonies as a template, and thereby amplifying the DNA containing a region encoding sorbitol dehydrogenase. After digesting the DNA with NdeI, the DNA was inserted into the NdeI site of pUTE500K' (Japanese Patent Application Laying-Open (kokai) No. 08-205861), an expression plasmid vector having a DNA sequence containing an expression region such as promoter, operator and a ribosome-binding site derived from E. *coli* lactose operon (refer to The Operon, p. 227, Cold Spring Harbor Laboratory, 1980), consequently affording a recombinant plasmid pSDH1 DNA.

In accordance with a method of D. M. Morrison (Methods in Enzymology, 68, p. 326-331, 1979), E. coli DH5α (available from Takara Shuzo Co., Ltd.) was transformed by use of the recombinant plasmid DNA pSDH1 thereby to obtain a transformant strain, *E. coli* DH5α (pSDH1). Incidentally, the *E. coli* DH5α (pSDH1) was deposited as FERM BP-7615 at the International Patent Organism Depository of the Independent Administrative Institution National Institute of Advanced Industrial Science and Technology.

After the obtained *E. coli* DH5α (pSDH1) was shake-cultured for 16 hours in a TY medium (1 % of bacto trypton, 0.5% of bacto yeast extract, 0.5% of NaCl, pH 7.0) containing 1mM isopropyl-β-D-thiogalactopyranoside, sorbitol dehydrogenase activity was measured and it was found to be 11.4 U/ml.

According to the present invention, sorbitol dehydrogenase can be produced efficiently.

**Claims**

1. A sorbitol dehydrogenase gene encoding the following protein (a) or (b):

   (a) a protein having an amino acid sequence shown in SEQ ID NO:1; or
   (b) a protein which consists of an amino acid sequence comprising one or more amino acid deletions, substitutions or additions relative to the amino acid sequence of (a), and has sorbitol dehydrogenase activity.

2. A sorbitol dehydrogenase gene comprising the following DNA (a) or (b):

   (a) a DNA having a nucleotide sequence shown in SEQ ID NO:2; or
   (b) a DNA encoding a protein which hybridizes with the DNA having a nucleotide sequence complementary to the DNA having a nucleotide sequence of (a) under stringent conditions, and has sorbitol dehydrogenase activity

3. A novel recombinant DNA, wherein the sorbitol dehydrogenase gene of claim 1 or 2 is inserted into a vector DNA.

4. A transformant or a transductant including the novel recombinant DNA of claim 3.

5. A method for producing sorbitol dehydrogenase, comprising the steps of:

   culturing the transformant or the transductant of claim 4 in a medium; and
   collecting sorbitol dehydrogenase from the culture.

SEQUENCE LISTING

<110> KIKKOMAN CORPORATION

<120> A SORBITOL DEHYDROGENASE GENE, A NOVEL RECOMBINANT DNA, AND A PROCESS FOR PRODUCING SORBITOL DEHYDROGENASE

<130> PH-1513

<160> 4

<210> 1

<211> 258

<212> PRT

<213> Pseudomonas sp. KS-E1806

<400> 1

```
Met Arg Leu Glu Asp Lys Val Ala Ile Leu Thr Gly Ala Ala Ser Gly
 1               5                   10                  15

Ile Gly Glu Ala Val Ala Gln Arg Tyr Leu Asp Glu Gly Ala Arg Cys
            20                  25                  30

Val Leu Val Asp Val Lys Pro Ala Gly Gly Ser Leu Ala Arg Leu Ile
            35                  40                  45

Glu Ala Asn Pro Gly Arg Ala Val Ala Val Thr Ala Asp Val Thr Arg
         50                  55                  60

Arg Asp Asp Ile Thr Arg Ile Val Ala Thr Ala Val Glu Arg Phe Gly
 65                  70                  75                  80

Gly Val Asp Ile Leu Phe Asn Asn Ala Ala Leu Phe Asp Met Arg Pro
                85                  90                  95

Leu Leu Asp Glu Ser Trp Asp Val Phe Asp Arg Leu Phe Ser Val Asn
               100                 105                 110

Val Lys Gly Leu Phe Phe Leu Met Gln Ala Val Ala Gln Arg Met Val
           115                 120                 125

Glu Gln Gly Arg Gly Gly Lys Ile Val Asn Met Ser Ser Gln Ala Gly
         130                 135                 140

Arg Arg Gly Glu Ala Leu Val Ser His Tyr Cys Ala Thr Lys Ala Ala
```

<pre>
            145               150               155               160
Val Ile Ser Tyr Thr Gln Ser Ala Ala Leu Ala Leu Ala Pro His Arg
                      165               170               175
Ile Asn Val Asn Gly Ile Ala Pro Gly Val Val Asp Thr Pro Met Trp
                  180               185               190
Glu Gln Val Asp Ala Leu Phe Ala Arg Tyr Glu Asn Arg Pro Leu Gly
              195               200               205
Glu Lys Lys Arg Leu Val Gly Glu Ala Val Pro Leu Gly Arg Met Gly
          210               215               220
Val Pro Gly Asp Leu Thr Gly Ala Ala Leu Phe Leu Ala Ser Ala Asp
225               230               235               240
Ala Asp Tyr Ile Thr Ala Gln Thr Leu Asn Val Asp Gly Gly Asn Trp
                  245               250               255
Met Ser
    258
</pre>

〈210〉 2

〈211〉 774

〈212〉 DNA

〈213〉 Pseudomonas sp.  KS-E1806

〈400〉 2

<pre>
gtg aga ctg gaa gac aag gtc gcg atc ctg acg ggc gcc gca agc ggc      48
atc ggc gag gcg gtc gca caa cgc tat ctg gac gag ggc gcg cgc tgc      96
gtg ctc gtc gac gtg aag ccg gca ggc ggc tcg ctc gcg cgg ctg atc     144
gag gcc aac ccg ggc cgc gcg gtg gcc gtc acg gcc gac gtc acg cgt     192
cgc gac gac atc acg cgg atc gtc gcc acg gcg gtc gag cgc ttc ggc     240
ggc gtc gac att ctg ttc aac aac gcg gcg ctg ttc gac atg cgt ccg     288
ctc ctc gat gaa tcc tgg gac gtg ttc gac cgg ctg ttc tcg gtc aac     336
gtg aaa ggg ctg ttc ttc ctg atg cag gcg gtt gcg caa cgg atg gtc     384
gag cag ggg cgc ggc ggc aag atc gtc aac atg tcg tcg cag gcc ggc     432
</pre>

```
cgt cgc ggc gag gcg ctc gtt tcg cac tac tgc gcg acc aag gcc gcg      480

gtg atc agc tat acg cag tcg gcc gcg ctc gcg ctc gcg ccg cac cgg      528

atc aac gtg aac ggc atc gcg ccg ggc gtg gtc gac acg ccg atg tgg      576

gag cag gtc gat gcg ctg ttc gcg cgc tac gag aac cgg ccg ctc ggc      624

gag aag aag cgg ctc gtc ggt gaa gcc gtg ccg ctc ggc cgc atg ggc      672

gtg ccg ggc gac ctg acg ggc gcc gcg ctg ttc ctc gcg tcg gcc gat      720

gcc gac tac atc acc gcc cag acg ttg aac gtc gat ggc ggc aac tgg      768

atg agc                                                              774
```

<210> 3

<211> 30

<212> DNA

<213> Artificial Sequence

<400> 3

```
aac cgg aga cag cat atg aga ctg gaa gac      30
```

<210> 4

<211> 30

<212> DNA

<213> Artificial Sequence

<400> 4

```
ctt gcg agc ggc cat atg tca gct cat cca      30
```